# EUROPEAN PATENT APPLICATION

(11) **EP 1 602 394 A1**
(43) Date of publication of application: **07.12.2005**
(21) Application number: 05076299.6
(22) Date of filing: 03.06.2005
(51) Int. Cl.: A61N 5/10

(54) **Device for storing and manipulating enriched low energy sources**

(30) Priority: 03.06.2004 NL 1026323
(71) Applicant: Isodose Control B.V., 3905 PE Veenendaal (NL)
(72) Inventor: van 't Hooft, Eric, 2930 Brasschaat (BE)
(74) Representative: Winckels, Johannes Hubertus F.

(57) **Abstract**

Device for storing and manipulating a radiation source, comprising: a safe for storing at least one capsule comprising a radioactive material, a transport element for moving the capsule through a transport tube connected to the safe and leading to an applicator, and a drive mechanism for driving the transport element. According to the invention, the capsule comprises an enriched low energy radiation source for administering gamma radiation in the range of 10-100 keV. For this purpose, a source is utilized having a sufficiently high specific activity for performing a high dose rate radiation treatment.

## Description

This invention relates to a device for storing and manipulating a radiation source, comprising: a safe for storing at least one capsule comprising a radioactive material, a transport element for moving the capsule through a transport tube connected to the safe and leading to an applicator, and a drive mechanism for driving the transport element.

With such devices, by means of the drive mechanism, for instance a transport wire wound onto a reel and connected with the capsule, a source can be brought to an applicator and halted or moved at one or more sites to locally effect a concentrated radiation in the body of a patient. Such devices are known and comprise a radioactive source of the isotope Cobalt 60, Cesium 137 or Iridium 192, which can be generally designated as "strong radiators".

Such isotopes require a highly shielded, 30-75 cm concrete treatment chamber to protect hospital personnel and other attendants.

The object of the invention is to provide a device with an isotope having a considerably lower emitted radiation energy, thereby allowing work to be done in standard treatment rooms or standard operating rooms with a shielding of 25 cm concrete at most.

This is achieved, according to the invention, by the use of the features of claim 1. According to the invention, therefore, a lower energy radiation source is used which has as an advantage that the shielding measures are considerably less strong because of the lesser penetrative power of the lower energy radiator. By enriching the lower energy radiation source, a higher dose rate can be delivered, which shortens the treatment without the radiation source unduly increasing in volume. It is noted that in itself the low energy radiation source as an application is known in the treatment of tumors, but the intensity of these radiators is too low to use a so-called high dose rate treatment (HDR treatment), whereby a tumor is exposed for a relatively short time to a high radiation intensity. In view of the specific use of the device, the known low energy radiators would necessitate an unduly long radiation time to deliver a sufficient dose.

Preferably, therefore, an isotope is used having a relatively lower energy with respect to the above-mentioned sources, such as ytterbium-169, iodine-125, palladium-103, thulium-170 or tungsten-181. According to the invention, these sources are enriched to a sufficient concentration to obtain sufficiently specific activity in a small capsule. This can be done, for instance, through an ultracentrifuge process.

Preferably, the source has been enriched such that the dose rate to be delivered comes to lie in the HDR range. This range is defined in the so-called ICRU reports (ICRU-38 en ICRU-58) known in the art. In a specific embodiment, the source is enriched such that the dose rate is at least 12 Gray/hour in a clinical specification spot, for instance at a distance of 1 cm from the source.

In a preferred embodiment, the source comprises palladium-103. The weight percentage of palladium-103 is then at least 3%. In another preferred embodiment, the source comprises iodine-125, preferably in a weight percentage of at least 15%. In a further preferred embodiment, the source comprises ytterbium-169, preferably in a weight percentage of ytterbium-169 of at least 0.5%. In another preferred embodiment the source may comprise tungsten-181 that is enriched to a weight percentage of at least 5%.

The capsule preferably has a diameter of at most 5 mm and a length of at most 50 mm.

Further features and advantages of the invention will be elucidated in more detail with reference to the drawing. In the drawing:
Fig. 1 shows a schematic set-up of the device;
Fig. 2 shows a schematic cross section of the source; and
Fig. 3 shows the results of a simulation of a dose absorbed by tissue.

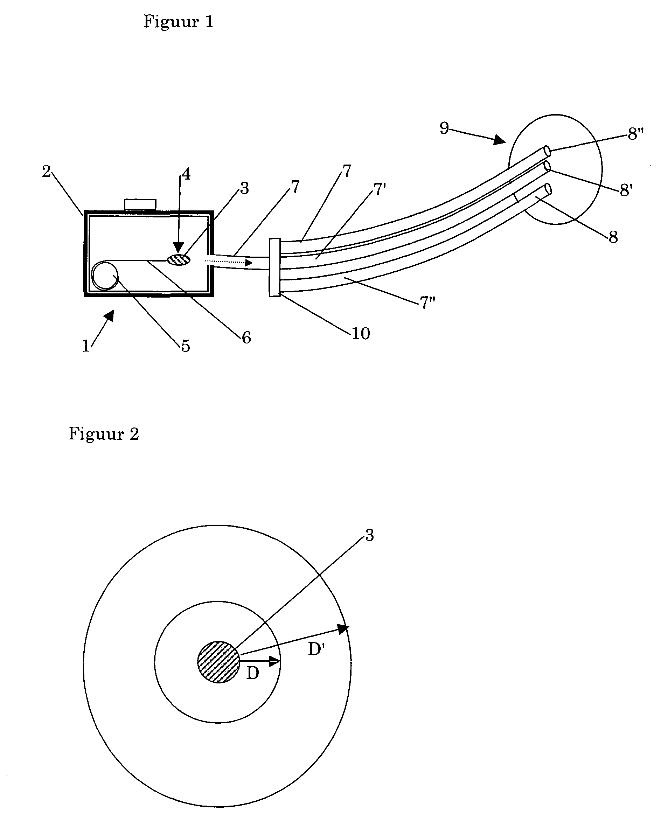

In Fig. 1 there is schematically represented an HDR device 1 provided with a safe 2 for storing at least one capsule 4 comprising a radioactive source 3, a transport wire 6 wound onto a reel 5 and connected with the capsule 4, to bring the capsule 4 via a transport tube 7 to an applicator 8 which has been introduced into tissue 9 to be irradiated. Such an HDR device is a device whereby a tissue is irradiated by one or more radiation sources with a relatively high activity, so that in a short time a high dose of radiation is absorbed by the tissue. According to the example, the capsule 4 has a diameter of at most 2,5 mm, preferably about 1 mm, for easy introduction via a tube into the body of a patient. For the types of radiations contemplated, greater diameters, while possible, are usually too great to allow positioning in the body without undesirable damage. Recommended with a view to easy transport of the capsule 4 is a length of at most ca. 50 mm, preferably even less, ca. 5 mm. As a consequence of this dimensioning of the capsule 4, the source 3 must have a sufficiently specific activity (i.e. a delivered radiation power per volume) to be effective for a radiation treatment. Too low a specific activity will then result in too long a treatment duration or can only be compensated by an mount of radiation material too large to be introduced into the tissue 9 via the transport tube 7. Fig. 1 depicts a variant of the device 1 in which a switch 10 is provided for connecting one or more transport tubes 7, 7', 7" simultaneously, which can be connected to one or more applicators 8, 8', 8". This enables simultaneous displacement of several capsules which may or may not be filled with radioactive material.

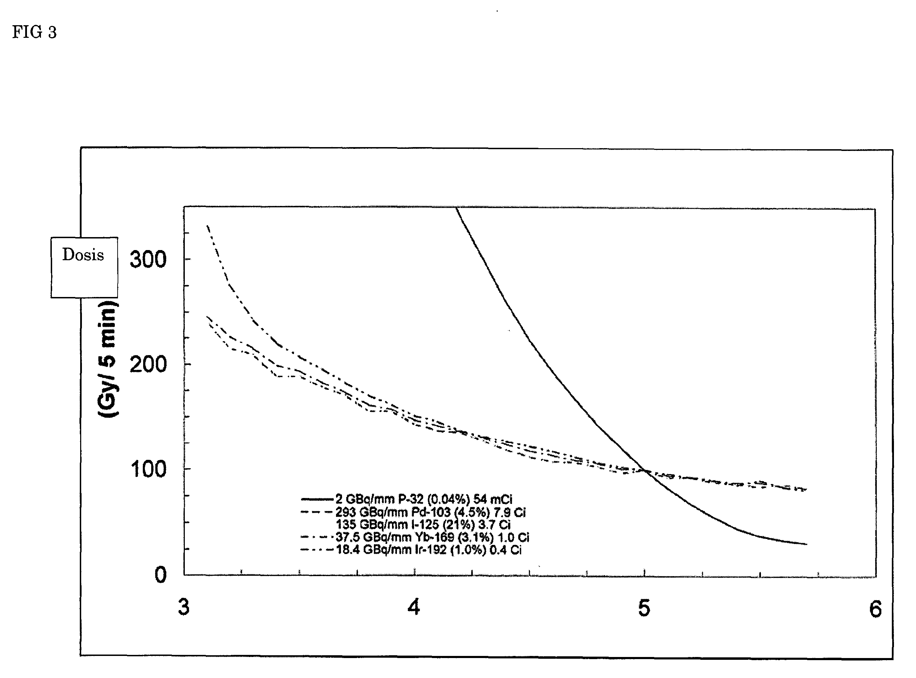

Fig. 2 schematically shows the simulation set-up for the table of Fig. 3, with the radiation source comprising a cylinder-shaped capsule having a diameter of 3 mm. At distances D, D', each time the total absorbed dose values per 5 minutes have been calculated over the total circumference of the cylinder (see fig. 3). A practical measure that is used for a desired radiation level for irradiating tissue is 50 Gray, while values of 10 Gray or more can be effective. The table in Fig. 3 shows the absorption values found for different low-energy enriched radiation sources, understood to include in particular gamma radiators in the energy range of 10-100 keV.

To achieve a desired absorption level, the table shows that for palladium-103, with a dose rate of 29 GBq/cm, a weight percentage of 4.5% is desired. Likewise, it has been found that for iodine-125 (135 GBq/mm) a minimum weight percentage of 21% is desired, and for ytterbium-169 (37.5 GBq/mm) a weight percentage of 2.5 %. Finally, for iridium-192 a desired weight percentage of 1% has been found.

The invention is not limited to the isotopes mentioned in the specification. It is also possible to use other isotopes of a sufficiently low energy, so that they can easily be shielded and they achieve a sufficiently high specific activity to fit into a small capsule. Such sources are understood to fall within the scope of the claims as defined in the following.

## Claims

1. A device for storing and manipulating a radiation source, comprising:
- a safe for storing at least one capsule comprising a radioactive material,
- a transport element for moving the capsule through a transport tube connected to the safe and leading to an applicator, and
- a drive mechanism for driving the transport element;
**characterized in that**
- the capsule comprises an HDR radiation source for emitting radiation substantially in the range of 10-100 keV.

2. A device according to claim 1, **characterized in that** the source is enriched such that a delivered dose rate comes to lie in the HDR range.

3. A device according to claim 1 or 2, **characterized in that** the source comprises palladium-103.

4. A device according to claim 3, **characterized in that** the weight percentage of palladium-103 is at least 3%.

5. A device according to claim 1 or 2, **characterized in that** the source comprises iodine-125.

6. A device according to claim 5, **characterized in that** the weight percentage of iodine-125 is at least 15%.

7. A device according to claim 1 or 2, **characterized in that** the source comprises ytterbium-169.

8. A device according to claim 7, **characterized in that** the weight percentage of ytterbium-169 is at least 0.5%.

9. A device according to claim 1, **characterized in that** the source contains thulium-170 (Tm¹⁷⁰).

10. A device according to claim 1, **characterized in that** the source contains tungsten-181 (W¹⁸¹).

11. A device according to claim 10, **characterized in that** the weight percentage of tungsten-181 (W¹⁸¹) is at least 5%.

12. A device according to at least one of the preceding claims, **characterized in that** the active source has a diameter of at most 5 mm.

13. A device according to at least one of the preceding claims, **characterized in that** the active source is at most 50 mm long.

14. A device according to at least one of the preceding claims, **characterized in that** the device is provided with a switch for simultaneously connecting one or more transport tubes which are connected to one or more applicators.

15. A device according to claim 1, **characterized in that** the device is provided with a switch capable of simultaneously moving several capsules which may or may not be filled with radioactive material.

16. A device according to any one of the preceding claims, **characterized in that** the device has several capsules, each filled with a different radioactive material.
